# EUROPÄISCHE PATENTSCHRIFT

(11) **EP 1 110 102 B1**
(45) Veröffentlichungstag und Bekanntmachung des Hinweises auf die Patenterteilung: **06.09.2006**
(21) Anmeldenummer: 99942841.0
(22) Anmeldetag: 12.08.1999
(51) Int. Cl.: G01S 15/89, A61B 8/00, A61B 5/02, A61B 8/12

(54) **VERFAHREN UND VORRICHTUNG ZUR AUFNAHME VON ULTRASCHALLBILDERN**
METHOD AND DEVICE FOR RECORDING ULTRASONIC IMAGES
PROCEDE ET DISPOSITIF POUR CAPTER DES IMAGES OBTENUES A L'AIDE D'ULTRASONS

(30) Priorität: 21.08.1998 DE 19838140
(43) Veröffentlichungstag der Anmeldung: 27.06.2001
(73) Patentinhaber: TomTec Imaging Systems GmbH, 85716 Unterschleissheim (DE)
(72) Erfinder: WALDINGER, Johannes, 85579 Neubiberg (DE); KAISER, Dietmar, 85368 Moosburg (DE); MUMM, Bernhard, 82291 Mammendorf (DE); MIYAMOTO, Kiyoji, Minato-ku, Tokyo 107 (JP)
(74) Vertreter: Müller, Hans-Jürgen
(86) Internationale Anmeldenummer: PCT/EP1999/005854
(87) Internationale Veröffentlichungsnummer: WO 2000/011495

(56) Entgegenhaltungen:
- EP-A- 0 514 584
- EP-A- 0 668 051
- EP-A- 0 736 284
- EP-A- 0 813 074
- WO-A-97/32277
- WO-A-98/24065
- US-A- 5 105 819
- US-A- 5 159 931

## Beschreibung

Die Erfindung betrifft ein Verfahren und eine Vorrichtung zur Aufnahme von Ultraschallbildern, insbesondere zur Aufnahme und Darstellung dreidimensionaler Ultraschallbilder in Echtzeit, nach der Ansprüche 1 und 11 sowie die Verwendung dieses Verfahrens bzw. dieser Vorrichtung nach dem Anspruch 15.

Derartige Verfahren und Vorrichtungen zur Aufnahme von Ultraschallbildern eines Objekts sind bekannt, bei denen ein Ultraschallsender Ultraschallwellen auf ein Objekt abstrahlt, während ein Ultraschallempfänger die von dem Objekt reflektierten Ultraschallwellen empfängt. Zur Aufnahme des Objekts wird der Ultraschallsender bzw. der Ultraschallempfänger entlang dem Objekt verfahren bzw. relativ zum Objekt rotiert, während einzelne Bild-Teilbereiche des Objekts aufgenommen werden. Diese Bild-Teilbereiche entsprechen im Normalfall einem zeilenweisen Scannvorgang, der das Objekt zeilenweise längs einer Aufnahmerichtung, in die der Ultraschallsender bzw. der Ultraschallempfänger verfahren wird, aufnimmt. Die im Ultraschallgerät erzeugten Bilder lassen sich bei den bekannten Ultraschallgeräten digital oder über einen Videoausgang in ein Nachverarbeitungsgerät bzw. in ein Datenverarbeitungsystem übernehmen. Dort können die Bilder gespeichert oder auch direkt nachverarbeitet werden.

Durch den Scannvorgang wird das zu untersuchende Objekt zeilenweise aufgenommen, d.h. es werden einzelne zueinander parallele "Schichten" bzw. zueinander rotationssymmetrische "Scheiben" des Objekts mit Ultraschallwellen beaufschlagt und die entsprechenden reflektierten Ultraschallwellen in dem Ultraschallgerät empfangen. Die empfangenen Ultraschallwellen werden in dem Datenverarbeitungsystem verarbeitet, so daß sich an einem Anzeigegerät ein Grauwertbild ergibt, wobei die einzelnen Grauwerte entsprechend stärker oder schwächer reflektierten Ultraschallwellen entsprechen. Diese Grauwerte liefern Informationen über die einzelnen Schichten bzw. Scheiben des Objekts, d.h. darüber, an welchen Stellen das Objekt beispielsweise Hohlräume oder höher verdichtete Materialbereiche aufweist.

Die einzelnen Schichten bzw. Scheiben des Objekts, d.h. die einzelnen Zeilenaufnahmen des Ultraschall-Scannvorganges werden in dem Datenverarbeitungssystem "übereinandergeschichtet", um dann am Anzeigegerät eine dreidimensionale Darstellung des Objekts zu erhalten. Die unterschiedlichen Abstände verschiedener Bereiche einer Schicht, d.h. die Lage von Hohlräumen oder stärker verdichteten Materialbereichen des Objekts relativ zu dem Ultraschallgerät, erhält man durch die Auswertung der Grauwertinformationen einer jeden Schicht.

Zwischen dem Ultraschallgerät und dem aufzunehmenden Objekt befindet sich ein Schallmedium, welches die Ausbreitung von Ultraschallwellen begünstigt. Dieses Schallmedium stellt sich in dem entsprechenden Grauwertbild durch einen einheitlichen Grauwert dar. Insbesondere lassen sich dadurch die Außenkonturen eines Objekts bestimmen, indem die ersten Grauwertveränderungen an der Grenze zwischen dem Schallmedium und dem zu untersuchenden Objekt in dem Grauwertbild detektiert werden und deren relative Abstände zu dem Ultraschallgerät gemessen werden.

Die Ultraschall-Verfahren benützen eine vordefinierte oder eine zu errechnende Grauwertstufe (Threshold), um im Bild Konturen zu finden. Die Kontureninformationen werden dann in einem Bild gespeichert und ergeben nach Auswertung der Entfernungen zwischen dem Ultraschallgerät bzw. dem Ultraschallkopf und den Außenkonturen des zu untersuchenden Objekts einen dreidimensionalen Bildeindruck.

Diese bekannten Ultraschallverfahren eignen sich beispielsweise zur Untersuchung eines Fötus innerhalb der Bauchhöhle der Mutter oder zur Detektion von Nierensteinen innerhalb des menschlichen Körpers. Zur Aufnahme des zu untersuchenden Objekts, welches bsp. innerhalb des menschlichen Körpers liegt, wird das Ultraschallgerät mittels eines Schallmediums, wie beispielsweise Gel, Öl oder Wasser mit der Hautoberfläche des menschlichen Körpers verbunden und entlang einer gewünschten Aufnahmerichtung bewegt bzw. rotiert, während in einheitlichen Zeit- oder räumlichen Abständen Ultraschallbilder aufgenommen werden. Der gesamte Scannvorgang erstreckt sich über einen bestimmten Bereich des menschlichen Körpers, wobei sukzessive während des Scannvorganges einzelne Schichten bzw. Scheiben des zu untersuchenden Objekts innerhalb des Körpers aufgenommen werden. Die einzelnen Ultraschallbilder werden in einem nachfolgenden Datenverarbeitungssystem wieder räumlich richtig aneinander gefügt, so daß sich durch "Aufschichten" der einzelnen Bilder ein vollständiges oder dreidimensionales Bild des Objekts ergibt. In diesem dreidimensionalen Bild können dann im Datenverarbeitungssystem "künstliche" zweidimensionale Bilder errechnet werden.

In der klinischen Praxis eingesetzte Ultraschallverfahren zur räumlichen Aufnahme von menschlichen Organen arbeiten derzeit tomographisch, d.h. das Volumen wird aus den einzelnen aufgenommenen Schichtebenen bzw. Scheiben zusammengesetzt. Bei der Transösophagealen Echokardigraphie wird beispielsweise dem Patienten durch die Speiseröhre eine schwenkbare endoskopische Sonde eingeführt. Der Ultraschallsensor ist als sogenanntes "phased array" in der Spitze der Sonde integriert. Dabei wird der Ultraschallkopf an der Spitze der Sonde entweder linear verschoben oder rotiert, so daß von jeder Winkeleinstellung des rotierenden Ultraschallkopfes bzw. von jeder verschobenen Position der Sonde eine Schicht des Organs abgetastet wird. Pro Schicht wird eine Bildsequenz, d.h. bsp. ein oder mehrere Bewegungszyklen des Organs, wie bsp. ein Herzzyklus, aufgenommen. Nach der Aufnahme einer solchen Sequenz wird der Ultraschallkopf bei der Rotation um ein gewünschtes Winkelinkrement mittels eines Motors, wie bsp. eines Schritt- oder Linearmotors, weitergedreht oder per Hand verschoben bzw. bei der linearen Verschiebung linear verschoben. Ein Datenverarbeitungssystem löst dann die nächste Aufnahmesequenz aus, wobei dieses Datenverarbeitungssystem sowohl die Daten des Elektrokardiogramms (EKG) als auch die Atmungs- oder Thoraxbewegung (Respirationsaufnahme) verarbeiten kann.

Durch die Kopplung des EKGs an die Aufnahmezeitpunkte wird versucht, jedes Bild einer Sequenz immer zu einem bestimmten Phasenpunkt während des Herzschlagzyklus aufzunehmen. Dadurch lassen sich bei bewegten Objekten bzw. Organen innerhalb von Lebewesen Sequenzen von dreidimensionalen Bildern erzeugen, die aneinandergereiht eine zeitabhängige dreidimensionale Darstellung des Organs ergeben. Die Organbewegung läßt sich dann wie in einem "Film" zyklisch betrachten.

Bei diesen Verfahren ist es besonders wichtig, daß die Abstände zwischen den einzelnen "Schichten", d.h. zwischen den einzelnen Bild-Teilbereichen des zu untersuchenden Objekts nahezu konstant sind, um eine Längenverzerrung des Gesamtbildes längs der Aufnahmerichtung, d.h. längs der Scannrichtung, zu vermeiden. Um eine gleichmäßige Aufnahmegeschwindigkeit, d.h. eine gleichmäßige Geschwindigkeit des Ultraschallkopfes längs der Aufnahmerichtung während des Scannvorganges zu erhalten, benutzen die Verfahren herkömmlicher Art entweder motorgesteuerte Mechaniken zum Bewegen des Ultraschallgeräts oder magnetische Positionsdetektoren bzw. optische Systeme, um jeweils die genaue Position des Ultraschallgeräts bezüglich der entsprechenden Aufnahme des Bild-Teilbereichs, d.h. der entsprechenden "Schicht" des zu untersuchenden Objekts zu detektieren. Durch die Detektion der genauen Position des Ultraschallgeräts während der Aufnahme einer solchen "Schicht" lassen sich später im Datenverarbeitungssystem die einzelnen Bild-Teilbereiche, d.h. die einzelnen "Schichten" des zu untersuchenden Objekts realitätstreu wieder zusammensetzen. Dadurch lassen sich bei den herkömmlichen Systemen Bildverzerrungen längs der Aufnahmerichtung vermeiden.

Aus der DE 38 29 603 A1 ist beispielsweise eine Ultraschallendoskopieeinrichtung bekannt, die zur Erzeugung multiplanarer Tomogramme über einen distalen Endbereich mit einem flexiblen Schlauch verfügt, in welchem ein Ultraschallwandler auf einem Gleitschlitten längsverschieblich bewegbar ist. Auch hier soll ein bestimmtes Volumen multiplanar genau abgetastet werden, um die dadurch erhaltenen Schnittbilder exakt räumlich rekonstruieren zu können. Dabei wird der Gleitschlitten mittels eines ihm zugeordneten Bowdenzugs durch einen Motor längs des distalen Endbereichs verschoben, um so die einzelnen Schichtbilder entsprechend aufzunehmen.

Aus der US 5 159 931 ist eine rotierende Ultraschallsonde bekannt, die von dem zu untersuchenden Organ einzelne rotationssymmetrische Scheiben aufnimmt, wobei hier die Sonde ebenfalls mittels eines Motors rotiert wird, der die Sonde gemäß einer spezifizierten Schrittfolge inkremental rotiert, so daß zu jeder aufgenommenen Schichtebene bzw. zu jeder Scheibe die genaue Position bekannt ist, um später im Datenverarbeitungssystem die einzelnen Bild-Teilbereiche der Rotationsaufnahmen einander zuordnen zu können.

Aus der EP 0 668 051 A2 ist ein Verfahren zur Aufnahme von Ultraschallbildern eines Objekts bekannt, indem ein manuell führbarer Ultraschallkopf Ultraschallaufnahmen des zu untersuchenden Objekts erzeugt. Zur Positionsbestimmung des Ultraschallkopfes verfügt dieser über Beschleunigungssensoren, die die Beschleunigung des Ultraschallkopfes in den 3 translatorischen und den 3 rotatorischen Bewegungsrichtungen detektieren. Durch Integration der Beschleunigung über die Zeit lässt sich die Geschwindigkeit und letztlich auch der zurückgelegte Weg des Ultraschallkopfes und somit die Position des Ultraschallkopfes bei der Aufnahme der Ultraschallbilder berechnen.

Diese herkömmlichen Systeme weisen den Nachteil auf, daß die entsprechenden Ultraschallgeräte sehr aufwendig und kostenintensiv produziert werden müssen, das Aufnahmeverfahren sehr umständlich zu handhaben ist sowie die Bedienperson, also beispielsweise der Arzt bei der Untersuchung eines Herzens, mit langen Aufnahme- und Nachverarbeitungszeiten zu rechnen hat.

Die bekannten motorgesteuerten Aufnahmesysteme mit Motoren, die sowohl parallel als auch mittels eines sogenannten "Sweep" oder mittels einer Rotation des Ultraschallkopfes die entsprechenden Organe aufnehmen, bedingen darüber hinaus eine ständige Kalibrierung der entsprechenden Motoren, eine Motorsteuerung sowie eine Energiezufuhr bis in den Organbereich von Lebewesen, in denen sich die Motoren bewegen, was Gefahrenrisiken für den Patienten beinhaltet. Auch die bekannten Systeme mit Positionssensoren (vergleiche beispielsweise DE 196 08 971 A1) benötigen einen aufwendigen apparativen Aufwand sowie eine Kalibrierung der Systeme sowie ggf. eine sogenannte "Reset"-Position, um die Ausgangslage des Ultraschallwandlers zu Beginn der Ultraschallaufnahme festzustellen.

Der Erfindung liegt die Aufgabe zugrunde, die Verfahren und Vorrichtungen der genannten Art dahingehend zu verbessern, daß motorgesteuerte Mechaniken oder Positionserkennungen des Ultraschallkopfes entbehrlich sind und dabei ein einfaches, kostengünstiges und leicht zu handhabendes Ultraschallverfahren bzw. Ultraschallgerät anzugeben.

Die Erfindung löst die genannte Aufgabe durch die in den Kennzeichenteilen der Ansprüche 1 und 11 genannten Merkmale. Vorteilhafte Ausgestaltungen der Erfindung sind in den jeweiligen Unteransprüchen gekennzeichnet; eine Verwendung der erfindungsgemäßen Vorrichtung bzw. des entsprechenden Verfahrens ist im Anspruch 15 gekennzeichnet.

Gemäß der Erfindung wird ein zu untersuchendes Objekt, welches sich beispielsweise in einem unzugänglichen Körper bzw. Körperbereich befindet, durch ein Ultraschallgerät aufgenommen. Dabei wird der Ultraschallkopf, welcher vorteilhafterweise den Ultraschallsender und den Ultraschallempfänger enthält, längs einer Aufnahmerichtung bewegt, rotiert bzw. fächerartig geschwenkt, während einzelne Bild-Teilbereiche des Objekts, d.h. einzelne "Schichten" bzw. Scheiben des Objekts aufgenommen werden. Diese Schichten werden in einem Datenverarbeitungssystem gespeichert und anhand der oben beschriebenen Grauwertanalyse ausgewertet. Anschließend werden die Schichten anhand der aufgenommenen Reihenfolge wieder zusammengesetzt, so daß sich ein dreidimensionales Ultraschallbild des zu untersuchenden Objekts ergibt.

Das resultierende Bild kann zwei- oder dreidimensional dargestellt werden, ev. sind auch durch die Aneinanderreihung und Darstellung einer Vielzahl dieser zwei- oder dreidimensionalen Bilder bewegte Bilder, sogenannte vierdimensionale Bilder, möglich. Die Bilder können auch farblich gekennzeichnet werden, um einen für die Bedienperson leichter erkennbaren Eindruck des zu untersuchenden Objekts zu erreichen. Zusätzlich zu diesen Grauwertinformationen (B-Mode) können auch die auf dem Dopplerprinzip basierenden Farb-, Bewegungs-, oder Flußinformationen ("Colourflow") oder jede andere zusätzliche Bildinformation in der gleichen Weise erfaßt werden. Dabei werden von einer Stelle des Objekts, welches sich bewegt, mehrere Aufnahmen erstellt, wobei die entsprechenden Aufnahmen (eine Vielzahl von Bild-Teilbereichen) eines Bewegungszustandes des Objekts jeweils zu einem resultierenden Bild zusammengesetzt werden. Die Vielzahl von resultierenden Bildern ergeben dann in Abfolge eine bewegte Darstellung des Objekts (Filmprojektion).

Um die genaue Reihenfolge der einzelnen Schichten bzw. Scheiben der einzelnen Bild-Teilbereiche festzustellen und um insbesondere die jeweiligen Abstände zwischen den einzelnen Schichtebenen festzustellen, wird die Bewegung des Ultraschallsenders bzw. des Ultraschallempfängers mittels eines Detektors schrittweise erfaßt und den einzelnen Bild-Teilbereichen zugeordnet. Dabei kann der Ultraschallkopf durch eine manuelle Bewegung beispielsweise des Arztes entlang dem Objekt verschoben bzw. rotiert werden, während ein Inkrementalgeber als Detektor zur schrittweisen Erfassung der Bewegung dient.

Vorteilhafterweise wird der Ultraschallsender bzw. der Ultraschallempfänger längs der Aufnahmerichtung mit gleichmäßiger Geschwindigkeit verfahren bzw. rotiert, um Schnittebenen mit in etwa gleichem Abstand zueinander zu erhalten, auch wenn dies aufgrund des Inkrementalgebers an sich nicht notwendig wäre. Die Abstände der einzelnen Bild-Teilbereiche untereinander werden durch die Auswertung der schrittweise erfaßten Positionen des Ultraschallsensors bzw. des Ultraschallempfängers bestimmt, die zuvor den einzelnen Bild-Teilbereichen zugeordnet wurden. So ist es zweckmäßig, bei der Aufnahme von Objekten den Ultraschallkopf gleichmäßig zu verfahren bzw. zu rotieren, wobei über Bilddatenleitungen die entsprechenden Bild-Teilbereiche an ein Datenverarbeitungssystem geleitet oder im Ultraschallgerät zwischengespeichert werden. Gleichzeitig übermittelt der Inkrementalgeber bzw. der Detektor die einzelnen Schrittweiten der Bewegung bzw. der Rotation des Ultraschallkopfes, die dann entsprechen der verstrichenen Zeit den einzelnen Bild-Teilbereichen zugeordnet werden. Dabei ist es auch möglich, die entsprechenden Positionen des Ultraschallkopfes, die über den Inkrementalgeber bestimmt werden, zusammen als sogenannte "header" an jenes Datenpaket anzuhängen, welches einem einzelnen Bild-Teilbereich entspricht.

Insbesondere ist dabei die Anzahl der schrittweise erfaßten Positionen in etwa gleich hoch oder sogar höher als die Anzahl der aufgenommenen Bild-Teilbereiche, um sicherzustellen, daß für jeden Bild-Teilbereich mindestens eine Position des Ultraschallkopfes zur Verfügung steht. Da die Aufnahme der einzelnen Bild-Teilbereiche und die Erfassung der Positionen mittels des Inkrementalgebers bei diesem sehr einfachen System meist nicht synchronisiert ist, empfiehlt sich die Detektion einer Vielzahl von unterschiedlichen Positionen, so daß zu jedem Bild-Teilbereich mit großer Annäherung auch die entsprechend exakte Position detektiert und diesem zugeordnet werden kann.

Für die Aufnahme von bewegten Objekten beispielsweise eines Herzens werden zu den Aufnahmen der einzelnen Bild-Teilbereiche die Bewegungszustände bzw. die absoluten oder relativen (zu einem Ereignis, wie z.B. der EKG-R-Zacke) Zeitstempel eines bewegten Objekts erfaßt, um dann die schrittweise erfaßten Positionen und die einzelnen Bild-Teilbereiche den Bewegungszuständen des Objekts zuzuordnen.

Anschließend werden die schrittweise erfaßten Positionen und Zeitstempel den Aufnahmen der einzelnen Bild-Teilbereiche wie oben beschrieben zugeordnet und diese entsprechend den Bewegungszuständen des Objekts zusammengesetzt und dargestellt. Dadurch ergeben sich die bereits erwähnten "vierdimensionalen" Bilddarstellungen. Mit Vorteil werden dabei die Zeitpunkte zur Aufnahme von einzelnen Bild-Teilbereichen des bewegten Objekts durch die Bewegungsabläufe des Objekts selbst gesteuert. Dazu dienen beispielsweise Signale des Elektrokardiogramms, der Atmung, der Magenbewegung, der Perestaltik der Speiseröhre oder einer Kombination aus diesen Signalen des Lebewesens, um die Zeitpunkte zur Aufnahme der einzelnen Bild-Teilbereiche festzulegen. Der Ultraschallsender bzw. der Ultraschallempfänger kann dabei linear, zirkular, bogenförmig oder freihand entlang dem Objekt verfahren werden, wobei je nach Bewegung geeignete Inkrementalgeber bzw. Detektoren angeordnet werden, um die Bewegung des Ultraschallkopfes schrittweise zu erfassen. Die einzelnen Bereiche des Objekts, die entsprechend den aufgenommenen Grauwertbildern und/oder Farbinformationen unterschiedliche Intensitäten aufweisen bzw. unterschiedlich bewegte Bereiche des Objekts, können bei der Darstellung farblich gekennzeichnet werden, um es dem Arzt zu erleichtern, die einzelnen Bereiche des Organs zu detektieren.

So können die Bilder, die aus den einzelnen Bild-Teilbereichen gebildet werden, während der Aufnahme des Objekts in Echtzeit verarbeitet und/oder angezeigt werden, so daß der Arzt das Organ manuell aufnehmen kann, um so beispielsweise den Fortschritt bei einer Operation besser beobachten zu können.

Als Inkrementalgeber zur schrittweisen Erfassung der Bewegung dienen mechanische, optische oder elektromagnetische Bewegungssensoren, die im Stand der Technik bereits bekannt sind. Beispiele dafür sind die von einer "Computermaus" bekannten Systeme zur Detektion der Bewegung der Maus mittels optischer Sensoren oder kugelförmiger Detektoren, die die Bewegung des Sensors in zwei Dimensionen detektieren. Darüber hinaus können rotationssymmetrische Inkrementalgeber die lineare Bewegung eines Ultraschallkopfes detektieren. Wird der Ultraschallkopf frei im Raum bewegt, eignen sich entsprechend gestaltete Mechaniken, um sämtliche Freiheitsgrade des Ultraschallkopfes inkremental zu erfassen. Besonders zweckmäßig ist es auch, den Inkrementalgeber in dem Ultraschallkopf zu integrieren und geeignete Meßabnehmer bzw. Meßgeber vorzusehen, die die Bewegung des Ultraschallkopfes in Relation zu seiner Umgebung schrittweise erfassen. Dies ist beispielsweise durch Kugelkörper an der Außenhaut des Ultraschallkopfes möglich, die bei der Bewegung des Ultraschallkopfes entlang einer Oberfläche diese Reltivbewegung schittweise detektieren.

Das erfindungsgemäße Verfahren bzw. die erfindungsgemäße Vorrichtung eignet sich insbesondere zur Aufnahme des Herzens eines Lebewesens unter Berücksichtigung der Bewegung des Herzens zur Darstellung eines bewegten, insbesondere vierdimensionalen Bildes. Dabei kann das Organ des Lebewesens oder Teile dieses Organs entweder transthorakal, transösophageal, abdominell, transrektal oder transvaginal mittels eines Ultraschallverfahrens aufgenommen werden; intravaskulär oder intraductal auch mittels eines Ultraschall- Katheterverfahrens. Auch Freihandaufnahmen eines durch den Arzt bewegten Ultraschallkopfes auf der Körperoberfläche des Patienten oder intraoperativ direkt auf oder in dem Organ sind ähnlich wie bei der Führung einer "Maus" eines Computers realisierbar.

Einige bevorzugte Ausgestaltungen der Erfindung werden anhand der nachfolgenden Zeichnungen näher erläutert. Dabei zeigen:
- Figur 1: eine schematische Darstellung der Aufnahme eines Herzens,
- Figur 2: ein rotationssymmetrischer Ultraschallwandler zur Aufnahme eines Herzens,
- Figur 3: ein Ultraschallwandler zur transthorakalen Echokardiographie,
- Figur 4: der Ultraschallwandler nach Figur 3 mit angeschlossenem Inkrementalgeber,
- Figur 5: ein Inkrementalgeber mit Ausgangssignal,
- Figur 6: ein linearer Ultraschallwandler zur parallelen Aufnahme des Herzens,
- Figur 7: die Schnittansicht des linearen Ultraschallwandlers nach Figur 6 mit Positionsdetektoren, und
- Figur 8: einen Inkrementalgeber zur Erfassung der linearen Bewegung des Ultraschallkopfes.

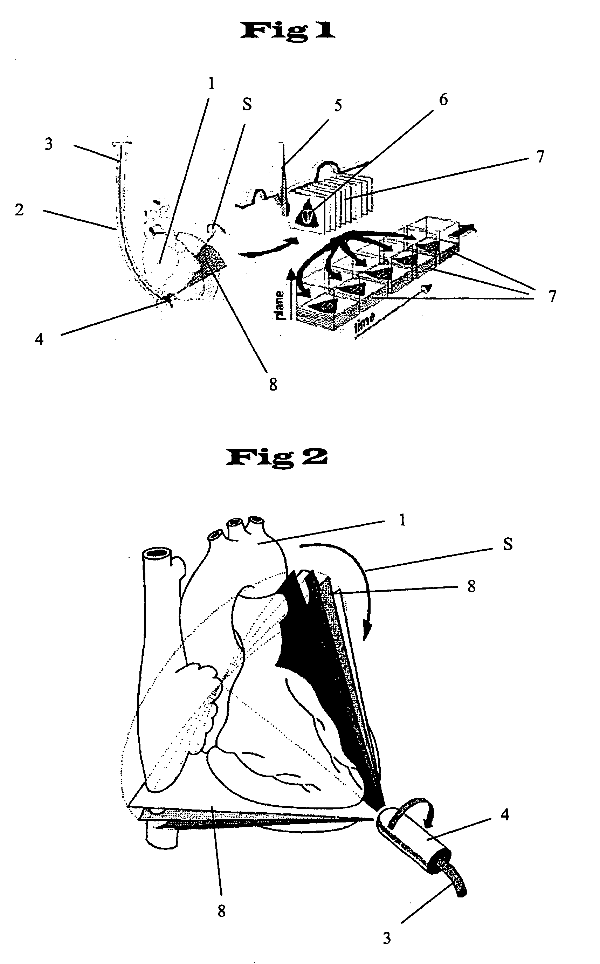

Figur 1 zeigt die schematische Ansicht zur Aufnahme eines Objekts 1, beispielsweise eines Herzens, welches transösophageal aufgenommen wird. Dabei wird ein Ultraschallkopf 4 durch die Speiseröhre 2 mittels eines flexiblen Schlauches 3 eingeführt, um parallele oder rotationssymmetrische Aufnahmen 7 des Objekts 1 aufzunehmen. Dabei rotiert z.B. eine aus Ultraschallstrahlen gebildete tomographische Ultraschallebene/bzw. -schnitt 8 rotationssymmetrisch in der Rotationsrichtung S.

Die einzelnen Bild-Teilbereiche 6, die untereinander den Abstand δx aufweisen, werden anhand eines EKG-Signals getriggert und entsprechend einander zugeordnet. Die einzelnen Bild-Teilbereiche 6 ergeben übereinandergeschichtet jeweils eine Aufnahme 7 zu einem bestimmten Bewegungszustand des Objekts 1. Die einzelnen Aufnahmen 7 können dann sukzessive an einem Bildschirm eines Datenverarbeitungssystems dargestellt werden, wodurch sich eine vierdimensionale, d.h. bewegte dreidimensionale Darstellung des Objektes 1 ergibt.

Figur 2 zeigt einen rotationssymmetrischen Ultraschallkopf 4, der an den flexiblen Schlauch 3 angeschlossen ist. Der Ultraschallkopf 4 sendet rotationssymmetrische aus Ultraschallstrahlen gebildete tomographische Ultraschallebenen-/bzw. - schnitte 8 aus, die in einer Rotationsrichtung S rotieren, um das Objekt 1 aufzunehmen. Die einzelnen Schichtebenen der rotationssymmetrischen Aufnahmen weisen dabei den Abstand δα auf.

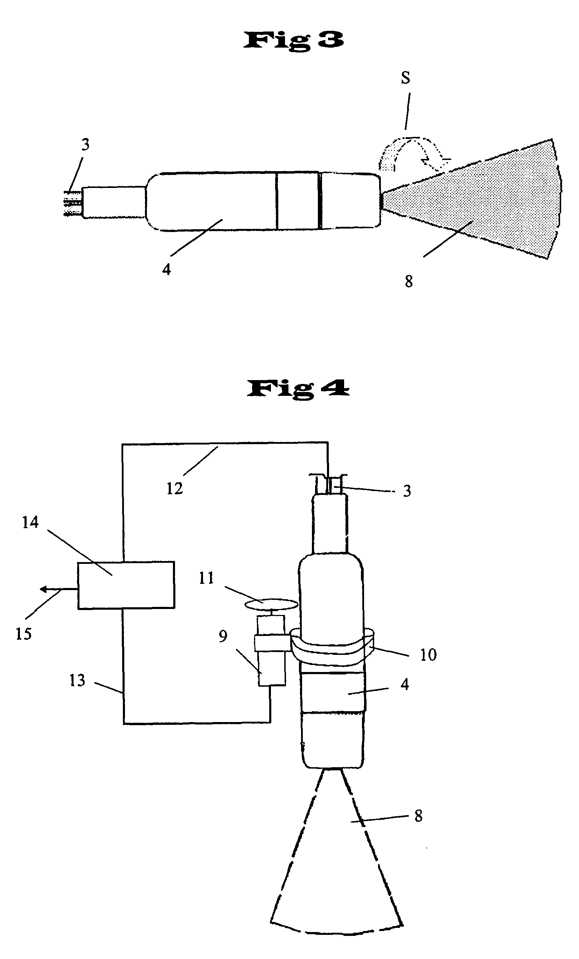

Figur 3 zeigt den Ultraschallkopf 4 nach Figur 2 mit dem angeschlossenen flexiblen Schlauch 3, der z.B. auch als Kabel ausgebildet sein kann, sowie die aus Ultraschallstrahlen gebildete tomographischen Ultraschallebenen-/ bzw. schnitte 8 und der Rotationsrichtung S.

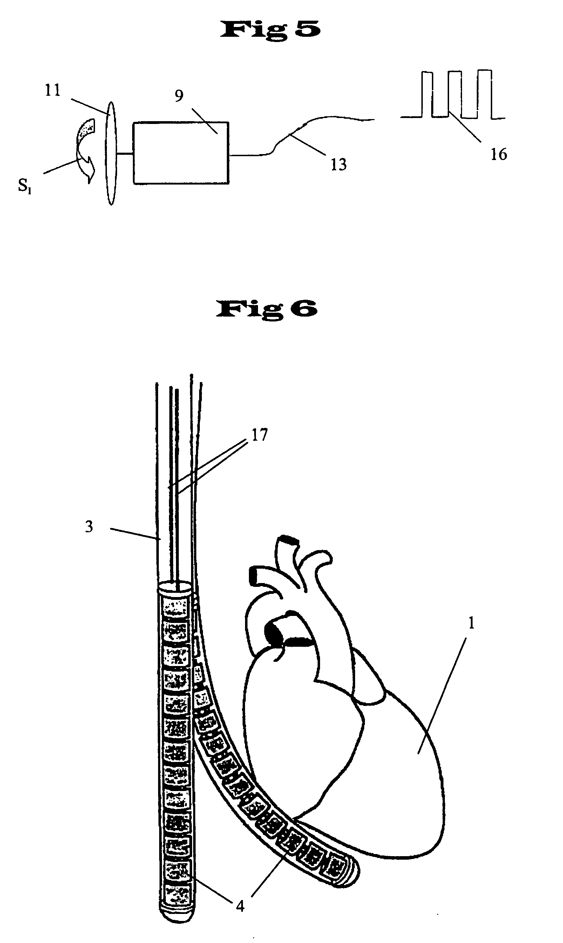

Figur 4 zeigt den Ultraschallkopf 4 nach Figur 3 mit dem flexiblen Schlauch 3, der sowohl Bilddatenleitungen 12 und gegebenenfalls auch Positionsdatenleitungen 13 aufnehmen kann. Am Ultraschallkopf 4, der rotationssymmetrische aus Ultraschallstrahlen gebildete, tomographische Ultraschallebenen-/bzw. -schnitte 8 aussendet, ist mittels einer Halterung 10 ein Inkrementalgeber 9 angeschlossen, der mittels eines Schrittgebers 11, beispielsweise eines kleinen Rades, schrittweise die Drehbewegung des Ultraschallkopfes 4 aufnimmt. Der Ultraschallkopf 4 wird dabei entweder durch Hand gedreht oder mittels eines Bowdenzuges, der ebenfalls im flexiblen Schlauch 3 zugeführt werden kann. Die Bilddatenleitungen 12 und die Positionsdatenleitungen 13 münden in ein Datenverarbeitungssystem 14, welches die einzelnen Bild-Teilbereiche 6 mittels der schrittweise erfaßten Positionen des Detektors 9 einander zuordnet und über eine Aufnahmendatenleitung 15 zur Bilddarstellung an ein weiteres Datenverarbeitungssystem weiterleitet.

Figur 5 zeigt den Detektor 9, d.h. den Inkrementalgeber mit angeschlossener Positionsdatenleitung 13 und Schrittgeber 11, der in Inkrementalrichtung S_{I} schrittweise die Bewegung des Ultraschallkopfes 4 bzw. des Ultraschallsenders oder Empfängers 18 aufnimmt. Die Positionsdatenleitung 13 liefert die entsprechenden Impulse 16 an das Datenverarbeitungssystem 14, das anhand der Zeitpunkte der aufgenommenen Bild-Teilbereiche 6 die einzelnen Positionen bzw. Impulse 16 des Detektors 9 den Bild-Teilbereichen 6 zuordnet. Zusätzlich zu den Bewegungsimpulsen 16 kann auch die Bewegungsrichtung S über ein zweites Signal des Gebers dem Datenverarbeitungssystem 14 zugeführt werden.

Figur 6 zeigt eine andere Ausführungsform eines linearen Ultraschallkopfes 4, der innerhalb des flexiblen Schlauchs 3 an den Rand bzw. in die Nähe des Objektes 1, beispielsweise eines Herzens, geführt wird. Zur Führung, d.h. zum Ein- und Ausführen des Ultraschallkopfes 4 dienen Zugschnüre 17, die innerhalb des flexiblen Schlauchs 3 zur mechanischen Ansteuerung des Ultraschallkopfes 4 dienen. In den vorangegangenen Beispielen könnte statt der Zugschnüre 17 auch ein Bowdenzug oder andere Vorrichtungen wie z.B. eine mechanische Welle vorgesehen sein, der innerhalb des flexiblen Schlauchs 3 den Ultraschallkopf 4 rotieren oder gleiten läßt. In Figur 6 dargestellt ist jedoch ein linearer Ultraschallkopf 4, d.h. ein längsverschieblicher Ultraschallkopf 4, der mittels der Zugschnüre 17 langsam von unten nach oben oder vice versa bewegt wird.

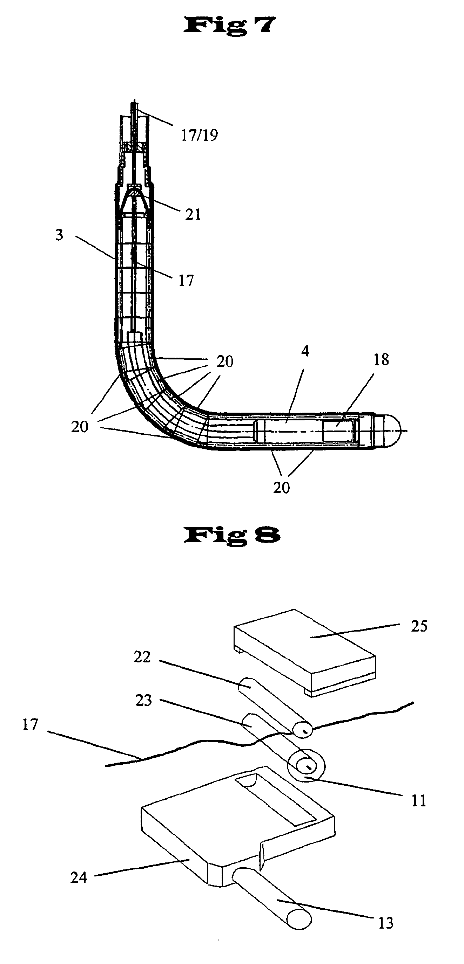

Figur 7 zeigt den Schnitt durch den linearen Ultraschallkopf 4 nach Figur 6 mit dem entsprechenden Ultraschallsender bzw. Empfänger 18 an der Spitze des Ultraschallkopfes 4 innerhalb des flexiblen Schlauchs 3. Bewegungssensoren 20, die sowohl optisch als auch mechanisch oder elektromagnetisch sein können, detektieren eine lineare Bewegung des Ultraschallkopfes 4 mittels der Zugschnüre 17, die nach oben durch den flexiblen Schlauch 3 geführt werden. Die Positionssignale, d.h. die schrittweise Erfassung der linearen Bewegung des Ultraschallkopfes 4 durch die Bewegungssensoren 20 werden in einem Signalkoppler 21 in eine Signalleitung 19 eingespeist.

Diese Signalleitung 19 kann sowohl ein Lichtleiter sein, der die optischen Positionssignale der Bewegungssensoren 20 an das Datenverarbeitungssystem 14 weiterleitet oder aber auch eine elektrische Leitung, die elektromagnetisch detektierte Bewegungen des Ultraschallkopfes 4 weiterleitet. Die Signalleitung 19 kann auch aus einem mechanischen Draht bestehen, der mittels des Signalkopplers 21 mechanische Verschiebungen der Bewegungssensoren 20 durch den flexiblen Schlauch 3 nach außen weiterleitet.

Figur 8 zeigt eine weitere Ausführungsform eines Detektors 9 in Form einer Detektorwalze 23, die gegen eine Druckwalze 22 gepreßt wird, um die relative Bewegung der Zugschnüre 17, die zwischen der Detektorwalze 23 und der Druckwalze 22 hindurchgezogen werden, zu detektieren. Sowohl die Detektorwalze 23 als auch die Druckwalze 22 liegen dabei innerhalb eines Gehäuseunterteils 24 und eines Gehäuseoberteils 25, die die Rotationsbewegung der Detektorwalze 23 mittels des Schrittgebers 11 aufnehmen und die dadurch detektierte schrittweise Position der entsprechenden Zugschnur 17 über eine Positionsdatenleitung 13 an das Datenverarbeitungssystem 14 weiterleiten. Statt der Zugschnur 17 kann auch der Ultraschallkatheter selbst verwendet werden, der bei intraductalen oder intravaskulären Ultraschallaufnahmen zum Einsatz kommt.

Das erfindungsgemäße Verfahren erübrigt die Kalibrierung sowie die Vorsehung eines Motors (z.B. ein Schrittmotor) als solches. Darüber hinaus entfällt die Motorsteuerung zur Regelung des Schrittmotors sowie die Zuführung von Energie zur Ansteuerung des Motors. Ebenfalls erübrigen sich Positionssensoren zur Detektierung eines Ultraschallwandlers, der außerhalb des Körpers des Lebewesens zur Aufnahme von Organen am Körper des Lebewesens entlang verfahren wird. Die entsprechend erfindungsgemäßen Inkrementalgeber sind billig und können mit hoher Genauigkeit die jeweilige Position des Ultraschallkopfes 4 bzw. des Ultraschallsenders bzw. -empfängers 18 schrittweise detektieren. Die bereits bekannten Verfahren zur Triggerung der entsprechend aufgenommenen Bild-Teilbereiche 6 nach Atemlage oder Herzphase sind darüber hinaus ebenfalls mit diesem erfindungsgemäßen Verfahren bzw. der erfindungsgemäßen Vorrichtung durchführbar.

## Patentansprüche

1. Verfahren zur Aufnahme von Ultraschallbildern eines Objekts, indem mit einem Utraschallsender Ultraschallwellen auf das Objekt abgestrahlt werden, und
durch einen Ultraschallempfänger von dem Objekt reflektierte Ultraschallwellen empfangen werden,
wobei der Ultraschallsender und/oder der Ultraschallempfänger zur Aufnahme von einzelnen Bild-Teilbereichen des Objekts (1) entlang dem Objekt mittels einer manuellen Bewegung (S) verfahren und/oder relativ zum Objekt (1) zumindest teilweise mittels einer manuellen Bewegung (S) rotiert wird, und wobei die Positionen des Ultraschallsenders und/oder des Ultraschallempfängers (4, 18)) mittels eines Detektors (9, 23) erfaßt und den einzelnen Bild-Teilbereichen (6) zugeordnet werden, wobei
der Detektor (9, 23) am Ultraschallsender und/oder Ultraschallempfänger (4, 18) angebracht wird,
**dadurch gekennzeichnet,**
**daß** die manuelle Bewegung (S) mittels eines Inkrementalgebers als Detektor (9, 23) schrittweise erfaßt wird, und
**daß** die Positionen des Ultraschallsenders und/oder des Ultraschallempfängers (4, 18) relativ zum Objekt (1) und asynchron zu den Aufnahmen von einzelnen Bild-Teilbereichen des Objekts (1) erfaßt werden.

2. Verfahren nach Anspruch 1,
**dadurch gekennzeichnet,**
**daß** der Ultraschallsender und/oder der Ultraschallempfänger (4, 18) längs der Aufnahmerichtung mit gleichmäßiger Geschwindigkeit verfahren bzw. rotiert wird.

3. Verfahren nach einem der Ansprüche 1 oder 2,
**dadurch gekennzeichnet,**
**daß** die Abstände (δx, δα) einzelner Bild-Teilbereiche (6) untereinander durch die Auswertung der schrittweise erfassten Positionen des Ultraschallsenders und/oder des Ultraschallempfängers (4, 18) bestimmt werden, die den einzelnen Bild-Teilbereichen (6) zugeordnet wurden.

4. Verfahren nach einem der vorhergehenden Ansprüche,
**dadurch gekennzeichnet,**
**daß** die Anzahl der schrittweise erfaßten Positionen in etwa gleich hoch oder höher ist als die Anzahl der aufgenommenen Bild-Teilbereiche (6).

5. Verfahren nach einem der vorhergehenden Ansprüche,
**dadurch gekennzeichnet,**
**daß** zu den Aufnahmen (7) der einzelnen Bild-Teilbereiche (6) die Bewegungszustände eines bewegten Objekts (1) erfaßt werden,
**daß** die schrittweise erfaßten Positionen und die einzelnen Bild-Teilbereiche (6) den Bewegungszuständen des Objekts (1) zugeordnet werden, und
**daß** die schrittweise erfaßten Positionen den Aufnahmen (7) der einzelnen Bild-Teilbereiche (6) zugeordnet und diese entsprechend den Bewegungszuständen des Objekts (1) zusammengesetzt und dargestellt werden.

6. Verfahren nach Anspruch 5,
**dadurch gekennzeichnet,**
**daß** die Zeitpunkte zur Aufnahme von einzelnen Bild-Teilbereichen (6) des Objekts (1) durch die Bewegungsabläufe des Objekts (1) gesteuert werden.

7. Verfahren nach Anspruch 5 oder 6 zur Aufnahme eines menschlichen oder tierischen Organs,
**dadurch gekennzeichnet,**
**daß** die Zeitpunkte zur Aufnahme von einzelnen Bild-Teilbereichen (6) anhand von Signalen des Elektrokardiogramms (5), der Atmung, der Magenbewegung, der Perestaltik der Speiseröhre oder einer Kombination aus diesen Signalen des Lebewesens gesteuert werden.

8. Verfahren nach einem der vorhergehenden Ansprüche,
**dadurch gekennzeichnet,**
**daß** der Utraschallsender und/oder der Ultraschallempfänger (4, 18) linear, zirkular, bogenförmig oder freihand entlang dem Objekt (1) verfahren wird.

9. Verfahren nach einem der vorhergehenden Ansprüche,
**dadurch gekennzeichnet,**
**daß** zusätzlich zu Grauwertinformationen (B-Mode) einzelner Bereiche des Objekts (1) bei der Darstellung insbesondere unterschiedlich bewegter Bereiche des Objekts (1) Farb-, Bewegungs-, oder Flußinformationen oder andere zusätzliche Bildinformationen erfaßt werden.

10. Verfahren nach einem der vorhergehenden Ansprüche,
**dadurch gekennzeichnet,**
**daß** die Bilder, die aus den Bild-Teilbereichen (6) gebildet werden, während der Aufnahme des Objekts (1) in Echtzeit verarbeitet und/oder angezeigt werden.

11. Vorrichtung zur Aufnahme von Ultraschallbildern eines Objekts, mit
einem Ultraschallsender zur Abstrahlung von Ultraschallwellen auf das Objekt,
einem Ultraschallempfänger zum Empfang von von dem Objekt reflektierten Ultraschallwellen,
wobei der Ultraschallsender und/oder der Ultraschallempfänger (4, 18) zur Aufnahme von einzelnen Bild-Teilbereichen des Objekts (1) entlang dem Objekt (1) manuell verfahrbar und/oder relativ zum Objekt zumindest teilweise manuell rotierbar ist, und einem
Detektor (9, 23), der die Positionen des Ultraschallsenders und/oder des Ultraschallempfängers (4, 18) erfaßt und den einzelnen Bild-Teilbereichen (6) zuordnet, und wobei der Detektor (9, 23) am Ultraschallsender und/oder Ultraschallempfänger (4, 18) angebracht ist.
**dadurch gekennzeichnet,**
**daß** ein Inkrementalgeber als Detektor (9, 23) die manuelle Bewegung (S) schrittweise erfaßt, und
**daß** der Detektor (9, 23) die Positionen des Ultraschallsenders und/oder des Ultraschallempfängers (4, 18) relativ zum Objekt (1) und asynchron zu den Aufnahmen von einzelnen Bild-Teilbereichen des Objekts (1) erfaßt.

12. Vorrichtung nach Anspruch 11,
**dadurch gekennzeichnet,**
**daß** als Inkrementalgeber ein mechanischer, optischer oder elektromagnetischer Bewegungssensor (20) zur schrittweisen Erfassung der Bewegung (S) dient.

13. Vorrichtung nach einem der Ansprüche 11 oder 12,
**dadurch gekennzeichnet,**
**daß** mehrere Inkrementalgeber zur schrittweisen Erfassung der Bewegung (S) mit zwei bis sechs Freiheitsgraden eines Ultraschallkopfes (4) dienen.

14. Vorrichtung nach einem der Ansprüche 11 bis 13,
**dadurch gekennzeichnet,**
**daß** der Detektor (9, 23) im Ultraschallsender und/oder im Ultraschallempfänger (4, 18) bzw. im Ultraschallkopf integriert ist.

15. Verwendung eines Verfahrens nach einem der Ansprüche 1 bis 10 oder einer Vorrichtung nach einem der Ansprüche 11 bis 14 zur dreidimensionalen Aufnahme eines Organs, insbesondere des Herzens eines Lebewesens, unter Berücksichtigung der Bewegung des Herzens zur Darstellung eines bewegten Bildes.

## Claims

1. Method of recording ultrasound images of an object by radiating ultrasound waves on to the object with an ultrasound transmitter, and ultrasound waves reflected by the object are received by an ultrasound receiver, wherein the ultrasound transmitter and/or the ultrasound receiver for recording individual image partial areas of the object (1) is/are moved along the object by means of manual movement (S) and/or is/are rotated relative to the object (1) at least partly by manual movement (S), and wherein the positions of the ultrasound transmitter and/or of the ultrasound receiver (4, 18) are detected by means of a detector (9, 23) and are allocated to the individual image partial areas (6), wherein the detector (9, 23) is mounted on the ultrasound transmitter and/or ultrasound receiver (4, 18), **characterised in that** the manual movement (S) is detected stepwise by means of an incremental transducer as a detector (9, 23) and **in that** the positions of the ultrasound transmitter and/or of the ultrasound receiver (4, 18) are detected relative to the object (1) and asynchronously to the recording of individual image partial areas of the object.

2. Method according to claim 1, **characterised in that** the ultrasound transmitter and/or the ultrasound receiver (4, 18) are moved or rotated with uniform speed along the recording direction.

3. Method according to either of claims 1 or 2, **characterised in that** the distances (□x, □□..of individual image partial areas (6) from one another are determined by evaluating the positions detected stepwise of the ultrasound transmitter and/or ultrasound receiver (4, 18), which are allocated to the individual image partial areas (6).

4. Method according to one of the preceding claims, **characterised in that** the number of positions detected stepwise is roughly equal to or more than the number of image partial areas (6) recorded.

5. Method according to one of the preceding claims, **characterised in that** for the recordings (7) of the individual image partial areas (6), the movement states of a moving object (1) are detected, **in that** the positions detected stepwise and the individual image partial areas (6) are allocated to the movement states of the object (1), and **in that** the positions detected stepwise are allocated to the recordings (7) of individual image partial areas (6) and these are assembled and represented according to the movement states of the object (1).

6. Method according to claim 5, **characterised in that** the instants for recording individual image partial areas (6) of the object (1) are controlled by movement sequences of the object (1).

7. Method according to claim 5 or 6 for recording a human or animal organ, **characterised in that** the instants for recording individual image partial areas (6) are controlled by means of signals of the electrocardiogram (5), the breathing, the stomach movement, the peristalsis of the oesophagus or a combination of these signals of the living organism.

8. Method according to one of the preceding claims, **characterised in that** the ultrasound transmitter and/or the ultrasound receiver (4, 18) is moved in a line, in a circle, in a curve, or freehand along the object (1).

9. Method according to one of the preceding claims, **characterised in that** in additional to grey value data (B-mode) of individual areas of the object (1) in the representation in particular of differently moving areas of the object (1), colour, movement, or flow data or other additional image data are detected.

10. Method according to one of the preceding claims, **characterised in that** the images formed from the image partial areas (6) are processed and/or displayed in real time during recording of the object (1).

11. Apparatus for recording ultrasound images of an object, comprising an ultrasound transmitter for radiating ultrasound waves on to the object, an ultrasound receiver for receiving ultrasound waves reflected by the object, the ultrasound transmitter and/or ultrasound receiver (4, 18) being manually movable along the object (1) and/or at least partially manually rotatable relative to the object in order to record individual image partial areas of the object (1), and comprising a detector (9, 23) which detects the positions of the ultrasound transmitter and/or of the ultrasound receiver (4, 18) and allocates these to the individual image partial areas (6), and wherein the detector (9, 23) is mounted on the ultrasound transmitter and/or ultrasound receiver (4, 18), **characterised in that** an incremental transducer as a detector (9, 23) detects the manual movement (S) stepwise, and **in that** the detector (9, 23) detects the positions of the ultrasound transmitter and/or of the ultrasound receiver (4, 18) relative to the object (1) and asynchronously to the recordings of individual image partial areas of the object (1).

12. Apparatus according to claim 11, **characterised in that** as an incremental transducer a mechanical, optical or electromagnetic movement sensor (20) is used for detecting the movement (S) stepwise.

13. Apparatus according to either of claims 11 or 12, **characterised in that** plural incremental transducers are used for detecting the movement (S) stepwise, with two to six degrees of freedom of an ultrasound head (4).

14. Apparatus according to one of claims 11 to 13, **characterised in that** the detector (9, 23) is incorporated in the ultrasound transmitter and/or in the ultrasound receiver (4, 18) or in the ultrasound head respectively.

15. Use of a method according to one of claims 1 to 10 or of an apparatus according to one of claims 11 to 14 for the three-dimensional recording of an organ, in particular of the heart of a living organism, taking into account the movement of the heart for the representation of a moving image.

## Revendications

1. Procédé pour enregistrer des images ultrasonores d'un objet en faisant rayonner des ondes ultrasonores sur l'objet à l'aide d'un émetteur à ultrasons, et des ondes ultrasonores réfléchies par l'objet sont reçues par un récepteur à ultrasons, dans lequel l'émetteur à ultrasons et/ou le récepteur à ultrasons servant à enregistrer des zones partielles individuelles imagées de l'objet (1) est(sont) déplacés le long de l'objet au moyen d'un mouvement manuel (S) et/ou est(sont) soumis à une rotation par rapport à l'objet (1) tout au moins partiellement par un mouvement manuel (S), et dans lequel les positions de l'émetteur à ultrasons et/ou du récepteur à ultrasons (4, 18) sont détectées au moyen d'un détecteur (9, 23) et sont affectées aux zones partielles individuelles imagées (6), dans lequel le détecteur (9, 23) est monté sur l'émetteur à ultrasons et/ou le récepteur à ultrasons (4, 18), **caractérisé en ce que** le mouvement manuel (S) est détecté pas à pas au moyen d'un transducteur incrémentiel jouant le rôle de détecteur (9, 23) et **en ce que** les positions de l'émetteur à ultrasons et/ou du récepteur à ultrasons (4, 18) sont détectées par rapport à l'objet (1) et de façon asynchrone par rapport aux enregistrements des zones partielles individuelles imagées de l'objet.

2. Procédé, selon la revendication 1, **caractérisé en ce que** l'émetteur à ultrasons et/ou le récepteur à ultrasons (4, 18) sont soumis à un déplacement ou à une rotation ayant une vitesse uniforme le long de la direction d'enregistrement.

3. Procédé, selon l'une des revendications 1 ou 2, **caractérisé en ce que** les distances (□x, □□..des zones partielles individuelles imagées (6) les unes par rapport aux autres sont déterminées grâce à l'évaluation des positions détectées pas à pas de l'émetteur à ultrasons et/ou du récepteur à ultrasons (4, 18) lesquelles ont été affectées aux zones partielles individuelles imagées (6).

4. Procédé, selon l'une quelconque des revendications précédentes, **caractérisé en ce que** le nombre de positions détectées pas à pas est approximativement égal ou supérieur au nombre des zones partielles imagées (6) ayant été enregistrées.

5. Procédé, selon l'une quelconque des revendications précédentes, **caractérisé en ce que**, lors des enregistrements (7) des zones partielles individuelles imagées (6), les états de mouvement d'un objet en mouvement (1) sont détectés, **en ce que** les positions détectées pas à pas et les zones partielles individuelles imagées (6) sont affectées aux états de mouvement de l'objet (1), et **en ce que** les positions détectées pas à pas sont affectées aux enregistrements (7) des zones partielles individuelles imagées (6) et celles-ci sont assemblées et représentées en fonction des états de mouvement de l'objet (1).

6. Procédé, selon la revendication 5, **caractérisé en ce que** les moments de l'enregistrement des zones partielles individuelles imagées (6) de l'objet (1) sont commandés par les séquences de mouvement de l'objet (1).

7. Procédé, selon la revendication 5 ou 6, pour l'enregistrement d'un organe humain ou animal, **caractérisé en ce que** les moments de l'enregistrement des zones partielles individuelles imagées (6) sont commandés au moyen des signaux de l'électrocardiogramme (5), de la respiration, du mouvement de l'estomac, du péristaltisme de l'oesophage ou de la combinaison de ces signaux chez l'organisme vivant.

8. Procédé, selon l'une quelconque des revendications précédentes, **caractérisé en ce que** l'émetteur à ultrasons et/ou le récepteur à ultrasons (4, 18) est déplacé suivant une ligne, un cercle, une courbe ou une trajectoire libre le long de l'objet (1).

9. Procédé, selon l'une quelconque des revendications précédentes, **caractérisé en ce qu'**en plus des données de la valeur des gris (mode B) des zones individuelles de l'objet (1), lors de la représentation en particulier des zones de l'objet ayant différents mouvements, des données concernant la couleur, le mouvement ou le flux ou des données imagées supplémentaires sont détectées.

10. Procédé, selon l'une quelconque des revendications précédentes, **caractérisé en ce que** les images constituées à partir des zones partielles imagées (6) font l'objet d'un traitement et/ou d'un affichage en temps réel pendant l'enregistrement de l'objet (1).

11. Appareil servant à enregistrer des images ultrasonores d'un objet, comprenant un émetteur à ultrasons pour faire rayonner des ondes ultrasonores sur l'objet, un récepteur à ultrasons pour recevoir les ondes ultrasonores réfléchies par l'objet, l'émetteur à ultrasons et/ou le récepteur à ultrasons (4, 18) pouvant être déplacés manuellement le long de l'objet (1) et/ou pouvant être soumis à une rotation manuelle tout au moins partiellement par rapport à l'objet afin d'enregistrer des zones partielles individuelles imagées de l'objet (1), et comprenant un détecteur (9, 23) lequel détecte les positions de l'émetteur à ultrasons et/ou du récepteur à ultrasons (4, 18) et affecte celles-ci aux zones partielles individuelles imagées (6), et dans lequel le détecteur (9, 23) est monté sur l'émetteur à ultrasons et/ou le récepteur à ultrasons (4, 18), **caractérisé en ce qu'**un transducteur incrémentiel jouant le rôle de détecteur (9, 23) détecte le mouvement manuel (S) pas à pas, et **en ce que** le détecteur (9, 23) détecte les positions de l'émetteur à ultrasons et/ou du récepteur à ultrasons (4, 18) par rapport à l'objet (1) et de façon asynchrone par rapport aux enregistrements des zones partielles individuelles imagées de l'objet (1).

12. Appareil, selon la revendication 11, **caractérisé en ce qu'**un capteur de mouvement mécanique, optique ou électromagnétique (20), employé à titre de transducteur incrémentiel, sert à détecter le mouvement (S) pas à pas.

13. Appareil, selon l'une des revendications 11 ou 12, **caractérisé en ce que** plusieurs transducteurs incrémentiels sont utilisés pour détecter le mouvement (S) pas à pas, alors que la tête à ultrasons (4) a une liberté de deux à six degrés.

14. Appareil, selon l'une des revendications 11 à 13, **caractérisé en ce que** le détecteur (9, 23) est intégré à l'émetteur à ultrasons et/ou au récepteur à ultrasons (4, 18) ou bien à la tête à ultrasons respectivement.

15. Utilisation d'un procédé selon l'une des revendications 1 à 10, ou d'un appareil selon l'une des revendications 11 à 14, pour effectuer l'enregistrement tri-dimensionnel d'un organe, en particulier du coeur d'un organisme vivant, en tenant compte du mouvement du coeur pour la représentation d'une image en mouvement.
